# EUROPÄISCHE PATENTSCHRIFT

(11) **EP 1 314 983 B1**
(45) Veröffentlichungstag und Bekanntmachung des Hinweises auf die Patenterteilung: **04.08.2010**
(21) Anmeldenummer: 02025081.7
(22) Anmeldetag: 12.11.2002
(51) Int. Cl.: G01N 33/557, C12Q 1/26, G01N 33/58

(54) **Bioanalytische Messverfahren zur Bestimmung von Katalasen und Peroxidasen, deren Konjugaten, Substraten, Aktivatoren, und Inhibitoren**
Bioanalytical measuring method for catalases and peroxidases, their conjugates, substrates, activators and inhibitors
Metode de détermination bioanalytique de catalases et de peroxidases, leurs conjugues, substrats, activateurs et inhibiteurs

(30) Priorität: 12.11.2001 DE 10155160
(43) Veröffentlichungstag der Anmeldung: 28.05.2003
(73) Patentinhaber: Chromeon GmbH, 93053 Regensburg (DE)
(72) Erfinder: Wolfbeis, Otto, 93053 Regensburg (DE); Wu, Meng, 93047 Regensburg (DE); Lin, Zhihong, 93047 Regensburg (DE)
(74) Vertreter: Dey, Michael

(56) Entgegenhaltungen:
- EP-A- 1 239 049
- US-A- 5 958 715
- DIAMANDIS E P ET AL: "MULTIPLE FLUORESCENCE LABELING WITH EUROPIUM CHELATORS. APPLICATIONTO TIME-RESOLVED FLUOROIMMUNOASSAYS" ANALYTICAL CHEMISTRY, AMERICAN CHEMICAL SOCIETY. COLUMBUS, US, Bd. 61, Nr. 1, 1989, Seiten 48-53, XP000046914 ISSN: 0003-2700
- GUDGIN DICKSON E F ET AL: "TIME-RESOLVED DETECTION OF LANTHANIDE LUMINESCENCE FOR ULTRASENSITVE BIOANALYTICAL ASSAYS" JOURNAL OF PHOTOCHEMISTRY AND PHOTOBIOLOGY B: BIOLOGY, ELSEVIER SCIENCE S.A., BASEL, CH, Bd. 27, Nr. 1, 1995, Seiten 3-19, XP001018129 ISSN: 1011-1344
- LOPEZ E ET AL: "Europium(III) Trisbipyridine Cryptate Label for the Time-Resolved Fluorescence Detection of Polymerase Chain Reaction Products Fixed on a Solid Support" CLINICAL CHEMISTRY, AMERICAN ASSOCIATION FOR CLINICAL CHEMISTRY. WINSTON, US, Bd. 39, Nr. 2, 1993, Seiten 196-201, XP002125037 ISSN: 0009-9147
- MORTON R C ET AL: "STREPTAVIDIN-BASED MACROMOLECULAR COMPLEX LABELED WITH A EUROPIUM CHELATOR SUITABLE FOR TIME-RESOLVED FLUORESCENCE IMMUNOASSAY APPLICATIONS" ANALYTICAL CHEMISTRY, AMERICAN CHEMICAL SOCIETY. COLUMBUS, US, Bd. 62, Nr. 17, 1. September 1990 (1990-09-01), Seiten 1841-1845, XP000165501 ISSN: 0003-2700
- OSER A ET AL: "Sensitive non-radioactive dot-blot hybridization using DNA probes labelled with chelate group substituted psoralen and quantitative detection by europium ion fluorescence" NUCLEIC ACIDS RESEARCH, OXFORD UNIVERSITY PRESS, SURREY, GB, Bd. 16, Nr. 3, 1988, Seiten 1181-1196, XP002001451 ISSN: 0305-1048

## Beschreibung

Die Erfindung betrifft Bestimmungsverfahren unter Verwendung von Enzymen aus der Gruppe der Katalasen und Peroxidasen und unter Verwendung von optischen Indikatoren aus der Gruppe Europium-Liganden-Komplexe. Das neue Verfahren macht sich den überraschenden Befund zu Nutze, dass das von gewissen Komplexen des Europium(III)-Ions als Ligand gebundene Wasserstoffperoxid (H₂O₂) für enzymatische Reaktionen zur Verfügung steht. Der durch Enzyme bewirkte Verbrauch von Wasserstoffperoxid verursacht eine Änderung in den optischen, insbesondere lumineszenz-optischen Eigenschaften der Europium-Komplexe. Mit Hilfe der neuen Reagenzien sind Katalasen bzw. Peroxidasen, aber auch deren Substrate, deren Hemmer und Aktivatoren auf verbesserte Weise nachweisbar bzw. bestimmbar. Durch Verwendung von Katalasen bzw. Peroxidasen als Marker in immunologischen oder genetischen Nachweisverfahren sind auch Antigene und Nukleinsäure-Oligomere nachweisbar bzw. bestimmbar.

Der Vorteil der erfindungsgemäßen Indikatoren liegt in deren großen Selektivität, Stokes'-Verschiebungen und langen Abklingzeiten. Damit bietet sich die Möglichkeit, über eine zeitlich auflösende Messung eine störende Untergrundfluoreszenz zuerst abklingen zu lassen und die Fluoreszenz des Indikators EuTcWP, eines Komplexes aus Eu, Tc und Wasserstoffperoxid, erst danach zu bestimmen, was zu extrem niedrigen Nachweisgrenzen in der Bioanalytik führt.

Enzymatische Methoden unter Verwendung von Katalasen bzw. Peroxidasen spielen eine bedeutende Rolle in der Bioanalytik. Zu den häufigsten Fragestellungen gehörem zum einen der qualitative Nachweis der Anwesenheit eines bestimmten Enzyms, zum anderen die quantitative Bestimmung dessen Aktivität. Aber auch Enzymsubstrate können auf diese Weise bestimmt werden. Schließlich können auch Hemmer bzw. Aktivatoren von Enzymen bestimmt werden, indem man deren reaktionsverlangsamenden bzw. reaktionsbeschleunigenden Einfluss quantitativ erfasst. Eine Zusammensetzung findet sich im Buch Enzymatic Methods of Analysis von G.G. Guilbault, Pergamon Press, 1970.

Katalasen sind Enzyme, die Wasserstoffperoxid (H₂O₂, im Folgenden als WP bezeichnet) als Erstsubstrat abbauen. Sie sind im Enzymkatalog in Gruppe 1.11.1.6. zusammengefasst. Peroxidasen (POx) sind Enzyme, die neben WP ein zweites Substrat (z.B. ein Phenol) benötigen. Sie sind im Enzymkatalog in Gruppe 1.11.1.7 zusammengefasst. Phenolartige Verbindungen sind typische Substrate für Peroxidasen.

Katalasen spielen eine bedeutende Rolle in der Zersetzung des Zellgiftes WP und in der Zellregulation, z.B. in der Apoptose. Ihre Aktivität kann bestimmt werden, indem man die Zersetzung des WP über die Zeit bei 240 nm verfolgt, aber dies ist in vivo wegen der sehr starken Eigenabsorption allen biologischen Materials bei dieser Wellenlänge praktisch unmöglich. Einfache alternative direkte Verfahren sind nicht bekannt.

Peroxidasen (als solche, aber auch als Marker in Immunkomplexen bzw. in einem ELISA) können auf verschiedene Weise sichtbar gemacht werden, aber praktisch immer beruht das Prinzip darauf, dass Peroxidasen WP verbrauchen bzw. mit ihren Substraten farbige bzw. fluoreszente Produkte bilden. Somit kann die Aktivität einer Peroxidase durch Messung des Verbrauches an WP oder der Bildung von farbigen Produkten optisch nachgewiesen werden.

Peroxidasen spielen auch als Marker in immunologischen Nachweisverfahren eine bedeutende Rolle. Diese sind unter dem Namen ELISA (enzyme-linked immuno-sorbent assay) bekannt geworden. Typischerweise wird eine Peroxidase als Marker an einen Antikörper gebunden. Wenn sich aus Antigen und Antikörper ein Komplex bildet, kann man die darin enthaltene POx über die Messung der Enzymaktivität nachweisen. Sinngemäß ähnliche Verfahren sind für den Nachweis einer eingetretenen Hybridisierungsreaktion zwischen zwei Nukleinsäuresträngen bekannt.

Peroxidasen, die mit Substraten (z.B. 4-Chlor-1-naphthol) unlösliche gefärbte Produkte liefern, werden des Weiteren im sogenannten Immunblotting eingesetzt, da das gefärbte Endprodukt einen einfachen (visuellen) Nachweis einer Immunreaktion ermöglicht. Schließlich werden Peroxidasen auch als Marker in histochemischen Nachweisverfahren eingesetzt, da die mit bestimmten Substraten, z.B. 3-Amino-1-ethylcarbazol, gebildeten Produkte in der Histologie gut visualisierbar sind.

Man kann somit sagen, dass der Analytik von Katalasen bzw. Peroxidasen in biochemischen Nachweis- und Bestimmungsverfahren eine außerordentlich hohe Bedeutung zukommt.

Die Analytik der Katalase erfolgte bisher häufig folgendermaßen. Katalase zersetzt WP, wodurch dessen starke Absorption bei 240 nm abnimmt. Die Geschwindigkeit der Abnahme der Absorption ist ein direktes Maß für die herrschende Aktivität an Katalase. Dieses Verfahren ist unpraktisch und nur bei Lösungen anwendbar, deren Eigenabsorption bei 240 nm gering ist, was selten der Fall ist. Katalase kann auch dadurch bestimmt werden, dass man den verlangsamenden Einfluss der Katalase auf die Bildung farbiger Produkte aus WP und einem Substrat wie Aminosalicylsäure (oder einem anderen Phenol) unter dem Einfluss einer Peroxidase bestimmt. Da Katalase das WP zersetzt, wird die Reaktion stetig langsamer. Auch dieses Verfahren ist arbeitsaufwendig.

Das hier vorgestellte Verfahren ist viel einfacher. Es beruht auf der Zugabe eines stark fluoreszierenden Komplexes bestehend aus dem Eu(III)-Ion, einem organischen Liganden, insbesondere einer β-Dicarbonylverbindung und Wasserstoffperoxid (WP). Dieser Komplex wird durch Katalase und unter Verbrauch von WP in einen deutlich schwächer fluoreszierenden Komplex (ohne WP) überführt. Die Geschwindigkeit der Reaktion kann fluorimetrisch durch Anregung bei 350 - 430 nm und Messung der Fluoreszenz bei > 600 nm verfolgt werden.

Die Analytik der Peroxidase erfolgte bisher zumeist unter Einsatz organischer Reagenzien. Optische Bestimmungsverfahren für Peroxidasen (POx) beruhen auf der irreversiblen Oxidation gewisser organischer Stoffe zu gefärbten oder fluoreszenten Produkten. Besonders verbreitet sind die POx-Substrate o-Dianisidin, o-Phenylendiamin, Pyrogallol, Azino-bis(3-ethylbenzthiazolin-6-sulfonsäure (ABTS™). Sie alle bilden mit Peroxidase stark gefärbte Produkte, die photometrisch erfassbar sind.

Manche Phenole geben mit WP in Gegenwart von Peroxidase fluoreszente Dimere (Y. Hayashi et al., Anal. Chim. Acta 1986, Band 186, S. 131ff), die unter UV-Licht eine hellblaue Fluoreszenz zeigen. Ein neueres Reagenz dieser Art ist die Homovanillinsäure, wie von Wang, Schubert & Renneberg in *Sensors & Actuators* B28 (1995) 3-7 berichtet wird. Leider weisen alle biologischen Materialien unter UV-Anregung eine starke Eigenfluoreszenz auf. Zhou et al. beschreiben in Analytical Biochemistry 253 (1997) 162 ein stabiles nicht-fluoreszierendes Derivat des Resorufins (Amplex Red), das durch das Enzym Peroxidase zu einem grün fluoreszenten Produkt umgesetzt werden kann und zur Bestimmung der Aktivität von Peroxidasen eingesetzt werden kann.

Das hier beschriebene neue Verfahren ist den bisherigen Verfahren überlegen, da es auf der Zugabe nur eines Reagenzes beruht, nämlich des Komplexes aus Eu, einem organischen Liganden, z.B. Tetracyclin (Tc) und WP, also beispielsweise eines Systems aus EuTc und WP. Das WP in diesem stark fluoreszierenden System ist für enzymatische Oxidation verfügbar, und der Komplex EuTcWP wird durch die Katalase oder Peroxidase in den weit weniger stark fluoreszenten EuTc-Komplex überführt. Durch Messung der Abnahme der Fluoreszenz des Komplexes EuTcWP hat man somit eine einfache Möglichkeit in der Hand, die Aktivität einer Katalase oder POx nachzuweisen.

Peroxidase-Analytik unter Einsatz von Reagenzien aus der Gruppe der Europium-Reagenzien ist an sich bekannt. Die Vorteile der Verwendung von Eu-Reagenzien liegen in deren großten Stokes'-Verschiebung und in den langen Abklingzeiten (die eine zeitaufgelöste Messung ermöglichen). Meyer und Karst berichten in der DE-Offenlegungsschrift 198 13 247.6 (1998) und in Analyst 125 (2000) 1537-1538 über ein Verfahren zur Bestimmung von Glucose unter Verwendung der Enzyme Meerrettich-Peroxidase und Glucose-Oxidase (GOx) sowie der Reagenzien p-Hydroxyphenylpropionsäure und Tb(EDTA). Dessen Fluoreszenz klingt deutlich langsamer ab als die Untergrundfluoreszenz vieler biologischer Proben.

Glucose wurde z.B. bestimmt, indem man zur Untersuchungslösung als erstes Reagenz die Glucose-Oxidase (GOx) zugab, als zweites Reagenz die p-Hydroxyphenylpropionsäure (pHPPS) und als drittes Reagenz das Enzym Peroxidase. Das von der Oxidase gebildete WP reagiert unter dem Einfluss der Peroxidase mit dem pHPPS zu einem Dimer, das dann mit dem vierten Reagenz (einem Lanthaniden-Ion) einen lumineszenten Komplex bildet, dessen Fluoreszenz durch Zugabe des fünften Reagenzes (Cäsiumchlorid) verstärkt wird. Dieses Verfahren ist sehr empfindlich, aber wegen des Bedarfes von fünf Reagenzien umständlich.

Lanthanidenkomplexe wurden auch als Marker in Immuntests eingesetzt. Gewisse dreiwertige Ionen der Lanthanoiden-Elemente können als kovalente Marker in Lumineszenz-Immuntests verwendet werden. In einem (DELFIA™ genannten) Verfahren wird der Antikörper mit einem nicht fluoreszierenden Lanthanoiden-Ion markiert und nach der Bildung des Immunkomplexes mit zwei Reagenzien (Chelator und Mizellarbildner) versetzt, was zu einem deutlichen Anstieg der Fluoreszenzintensität führt. Das Verfahren wurde von I. Hemmilä in einem Übersichtsartikel mit dem Titel *Progress in Delayed Fluorescence Immunoassays* beschrieben, der im Buch Fluorescence Spectroscopy: Methods & Applications (Wolfbeis, O.S., Ed.; Springer Verlag, Heidelberg, 1993, S. 259-265) erschienen ist. Diamandis und Christopoulos geben in Anal. Chem. 62 (1990) 1149A ebenfalls eine Übersicht.

In einem alternativen Immuntest wird der Lanthaniden-Ligand (z.B. die Phenanthrolindicarbonsäure BCPDA) über eine Avidin-Biotin-Bindungsreaktion zugegeben, danach eine Lösung von Eu(III)-Nitrat. Nach Trocknen wird die Fluoreszenz ausgelesen. Eine Übersicht wurde von Evangelista et al. in Clinical Biochemistry 21 (1988) 173 verfasst. In neueren Verfahren werden auch Europium-markierte Nanopartikel (10 bis 500 nm Durchmesser) als Marker eingesetzt.

In all diesen Verfahren wird ein Lanthanoid eingesetzt, das als Marker angesehen werden kann und kovalent an ein Protein geknüpft werden muss. Im erfindungsgemäßen Verfahren wird kein kovalenter Marker eingesetzt, sondern ein neues Reagenz zugegeben, das durch eine Katalase oder Peroxidase zersetzt wird. Das neue Verfahren beruht zum anderen auf der Bestimmung einer Photolumineszenz und nicht auf der Bestimmung einer Chemilumineszenz, wie sie z.B. bei der Verwendung von Luminol zusammen mit Peroxidasen auftritt. Im Gegensatz zu chemilumineszenten Verfahren wird beim neuen Verfahren immer eine Anregungslichtquelle eingesetzt, die in der Lage ist, Licht zwischen 300 und 450 nm abzugeben.

Es ist bekannt, dass das Europium(III)-Ion mit bestimmten β-Dicarbonylverbindungen stark fluoreszierende Komplexe bildet. Dies gilt für eine Reihe von β-Dicarbonylverbindungen und für zahlreiche Chelatbildner, wie z.B. das EDTA. Des Weiteren wurde kürzlich von Y. Rakicioglu, J. H. Perrin und S.G. Schulman im J. Pharm. Biomed. Anal. 20:397-399 (1999) berichtet, dass die Analytik des Antibiotikums Tetracyclin verfeinert werden kann, wenn man das Eu(III)-Ion im Tetracyclin-Europium-Chelat mit Wasserstoffperoxid (WO)P) "oxidiert". Tatsächlich kann man das Antibiotikum auf diese Weise mit hoher Empfindlichkeit nachweisen.

Eine Aufgabe der Erfindung war es, ein verbessertes und insbesondere ein hoch sensitives Nachweisverfahren für Enzyme, beispielsweise für Katalasen oder Peroxidasen bereitzustellen.

Diese Aufgabe wird erfindungsgemäß gelöst durch ein Verfahren zum qualitativen oder quantitativen Nachweis eines Enzyms ausgewählt aus Katalasen oder Peroxidasen oder eines Substrats, Hemmers oder Aktivators davon in einer Probe, dadurch gekennzeichnet, dass man
(a) der Probe ein Reagenz zusetzt, das ein dreiwertiges Europium-Ion, mindestens einen organischen Liganden des Europium(III)-Ions und Wasserstoffperoxid enthält,
(b) der Probe gegebenenfalls ein Substrat der Peroxidase zusetzt und
(c) anhand von Änderungen der optischen Eigenschaften des in (a) eingesetzten Reagenzes das Enzym qualitativ nachweist oder quantitativ bestimmt.

Es wurde nun überraschenderweise gefunden, dass Chelate des Europium(III)-Ions mit organischen Liganden, insbesondere mit gewissen β-Dicarbonylverbindungen das Wasserstoffperoxid (WP) nur schwach gebunden haben können, denn in den entstehenden Produkten steht das eingesetzte WP erstaunlicherweise nach wie vor für enzymatische Reaktionen zur Verfügung. Das WP kann also keineswegs als Oxidationsmittel verbraucht worden sein, wie von Rakicioglu et al. berichtet worden war. Erfindungsgemäß wird dieser Befund dazu verwendet, um über die Unterschiede in den optischen Eigenschaften der Europium-Liganden-Komplexe mit bzw. ohne WP eine verbesserte enzymatische Analytik zu ermöglichen.

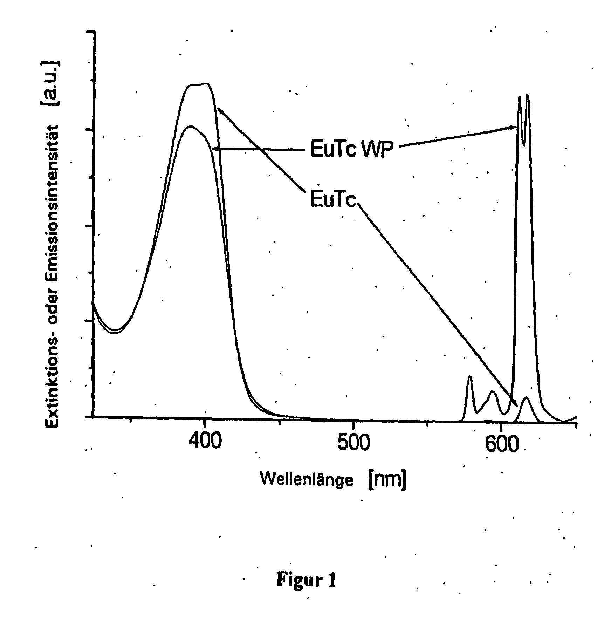

Die erfindungsgemäßen Reagenzien bilden sich durch Zugabe von WP zu den Komplexen des Europium(III)-Ions mit organischen Liganden, insbesondere mit β-Dicarbonylverbindungen, und weisen gegenüber den Komplexen ohne WP eine deutlich erhöhte Fluoreszenzintensität auf. Dies ist in Figur 1 dargestellt. Auch die Fluoreszenzabklingzeit ändert sich auf Zugabe von WP: Ohne WP liegt z.B. die Abklingzeit des EuTc-Komplexes bei etwa 13 µs, in Gegenwart von WP (also im EuTcWP-Komplex) bei etwa 9 µs.

Das erfindungsgemäße Verfahren kann zum qualitativen Nachweis eingesetzt werden, um festzustellen, ob überhaupt ein Enzym, dessen Substrat, Hemmer oder/und Aktivator in einer Probe vorhanden ist. Es ist aber auch zur quantitativen Bestimmung geeignet. Die nachzuweisenden Enzyme können beispielsweise in freier, immobilisierter oder konjugierter Form vorliegen. Bevorzugt handelt es sich bei der Probe um eine zu untersuchende Lösung, wobei man das Reagenz in Schritt (a) in fester oder gelöster Form zugibt. Das Reagenz ist bevorzugt ein Komplex bestehend aus einem dreiwertigen Europium-Ion, mindestens einem organischen Liganden des Europium(III)-Ions und Wasserstoffperoxid. Gemessen wird erfindungsgemäß die Änderung der optischen Eigenschaften des eingesetzten Reagenzes, insbesondere eine Änderung der Fluoreszenzoptischen Eigenschaften. Dabei wird Licht einer Anregungswellenlänge, vorzugsweise einer Wellenlänge zwischen 300 und 450 nm eingestrahlt und die Fluoreszenz bei einer zweiten Wellenlänge, der sogenannten Messwellenlänge, beispielsweise zwischen 550 und 700 nm bestimmt. In einer bevorzugten Ausführungsform erfolgt die Bestimmung, nachdem man zuerst eine gewisse Verzögerungszeit, insbesondere 1 µs bis 500 µs, bevorzugt 2 µs bis 40 µs zum Abklingen der kurzlebigen Untergrundfluoreszenz abwartet und dann die Messung startet. Es ist auch möglich 1 µs bis 5 µs, bevorzugt 2 µs bis 4 µs abzuwarten. Diese Messtechnik hat den Vorteil, dass die kurzlebige Unterfluoreszenz praktisch vollständig ausgeblendet werden kann.

Im Fall der Bestimmung einer Peroxidase wird der Lösung weiterhin vorzugsweise ein Substrat der Peroxidase zugesetzt.

Bevorzugte Ausführungsformen der Erfindung sind z.B. qualitative Nachweisverfahren und quantitative Bestimmungsverfahren für Katalasen in freier, immobilisierter bzw. konjugierter Form, sowie für deren Substrate, Hemmer und Aktivatoren, welche dadurch gekennzeichnet sind, dass man (a) einer zu untersuchenden Lösung ein festes oder gelöstes Reagenz zusetzt, das aus einem dreiwertigen Europium-Ion, zumindest einem organischen Liganden des Europium(III)-Ions und aus Wasserstoffperoxid besteht, (b) die durch eine Katalase bewirkte Änderung der optischen, insbesondere fluoreszenz-optischen Eigenschaften des Reagenzes misst, und (c) über die eingetretene Änderung der optischen Eigenschaften des Reagenzes die Katalase, deren Substrate, Hemmer oder Aktivatoren qualitativ nachweist oder quantitativ bestimmt.

Weitere bevorzugte Ausführungsformen sind qualitative Nachweisverfahren und quantitative Bestimmungsverfahren für Peroxidasen in freier, immobilisierter bzw. konjugierter Form, sowie für deren Substrate, Hemmer und Aktivatoren, welche dadurch gekennzeichnet sind, dass man (a) einer zu untersuchenden Lösung ein festes oder gelöstes Reagenz zusetzt, das aus einem dreiwertigen Europium-Ion, zumindest einem organischen Liganden des Europium(III)-Ions und aus Wasserstoffperoxid besteht, (b) der Lösung gegebenenfalls ein Substrat der Peroxidase zusetzt, (c) die durch eine Katalase bewirkte Änderung der optischen, insbesondere fluoreszenz-optischen Eigenschaften des Reagenzes misst, und (d) über die eingetretene Änderung der optischen Eigenschaften des Reagenzes die Peroxidase, deren Substrate, Hemmer oder Aktivatoren, qualitativ nachweist oder quantitativ bestimmt.

In einer anderen Ausführungsform liegt das Enzym, insbesondere eine Katalase oder Peroxidase an ein Biomolekül, vorzugsweise einen Antikörper, ein Antigen, eine DNA, eine RNA oder eine PNA oder an einen festen Träger konjugiert vor.

Bei der erfindungsgemäß zu untersuchenden Probe handelt es sich bevorzugt um eine Lösung und insbesondere um Blut, Serum, interstitielle Flüssigkeit, biologisches Gewebe, Nahrungsmittel, Getränke bzw. deren Vorprodukte, pflanzliche Produkte, Bioreaktorflüssigkeiten, Zellen oder Umweltproben.

Die erfindungsgemäßen Europium-Reagenzien besitzen bevorzugt die folgende chemische, nicht notwendigerweise stöchiometrische Zusammensetzung:

Eu - Lig - (H₂O₂)

worin
1. Eu für das dreiwertige Europium-Ion und
   Lig für zumindest einen Liganden der allgemeinen chemischen Struktur

   R¹-CO-C(R²) = C(X)-R³

   steht, worin
   maximal zwei der Reste R¹, R² oder R³ für H stehen können,
   X für OH, NHR⁴, NR⁴₂ stehen kann,
   R¹ bis R³ jeweils unabhängig voneinander H, einen linearen, verzweigten oder cyclischen, gesättigten oder einfach oder mehrfach ungesättigten Alkylrest, einen linearen, verzweigten oder cyclischen, gesättigten oder einfach oder mehrfach ungesättigten Alkanoylrest, einen Aroylrest, CF₃, einen gegebenenfalls substituierten (geeignete Substituenten sind z.B. Hydroxy, C₁-C₆-Alkoxy, Halogen oder Amino) Alkylrest oder Alkanoylrest, OH, NH₂, Alkylamino oder Dialkylamino, bedeutet,
   R⁴ für H, Alkyl oder Aryl stehen kann, wobei
   jeder der Reste R¹ bis R⁴ über einen (gegebenenfalls substituierten z.B. mit Hydroxy, C₁-C₆-Alkoxy, Halogen oder Amino) carbocyclischen bzw. heterocyclischen Ring mit einem der anderen Reste R¹ - R⁴ verbunden sein kann und
2. das molare Verhältnis von Eu zu Lig zwischen 30:1 und 1:5 und das molare Verhältnis von Eu zu WP zwischen 1:200 und 25:1 liegt.

Die hierin angegebenen Alkylreste bzw. Alkanoylreste enthalten vorzugsweise 1 bis 30 C-Atome, insbesondere 1 bis 8 C-Atome und besonders bevorzugt 1 bis 4 C-Atome. Die hierin angegebenen Arylreste bzw. Aroylreste enthalten vorzugsweise 4 bis 30, insbesondere 5 bis 20 und besonders bevorzugt 6 bis 15 C-Atome.

Das molare Verhältnis von Eu zu Lig liegt bevorzugt zwischen 20:1 und 1:3 und das molare Verhältnis von Eu zu WP liegt bevorzugt zwischen 1:100 und 10:1.

Die so erhaltenen Reagenzien können in fester oder gelöster Form vorliegen. Bevorzugte Liganden sind Benzoylaceton, Benzoyltrifluoroaceton, Dibenzoylmethan,Thenoyltrifluoroaceton,heterocyclische(ortho-Hydroxy)-Carbonsäuren, aromatische oder heterocyclische ortho-Hydroxyketone und deren Derivate, Hydroxychinone und teilweise hydrierte und substituierte Hydroxychinon-artige Verbindungen und annelierte Carbocyclen einschließlich des Tetracyclins und seiner Derivate.

Ein bevorzugtes Reagenz ist der Komplex aus Europium-Ion mit Tetracyclin und Wasserstoffperoxid (EuTcWP). Seine Fluoreszenzintensität ist abhängig von der Stöchiometrie zwischen Europium-Ion und Tetracyclin (typische Bandbreite 10:1 bis 1:10). Die Fluoreszenz des EuTcWP wird deutlich erniedrigt, wenn dessen WP durch Katalasen oder Peroxidasen verbraucht wird.

Das erfindungsgemäße Verfahren ermöglicht die Bestimmung verschiedener mit Enzymen in Beziehung stehender Parameter. So kann die Aktivität von gelösten oder konjugierten Enzymen aus der Gruppe der Katalasen und Peroxidasen nachgewiesen werden.

Zum Nachweis von Katalasen oder Peroxidasen setzt man dem zu untersuchenden System ein erfindungsgemäßes Reagenz zu, im Fall der Peroxidase zusätzlich auch ein Substrat, wie z.B. Phenol. Danach wird die Abnahme der Fluoreszenz bei einr Anregungswellenlänge von 330 bis 430 nm, insbesondere 350 bis 400 nm und einer Emissionswellenlänge von ca. 600 bis 630 nm detektiert bzw. über die Zeit verfolgt. Alternativ kann man auch die Zunahme der Abklingzeit bestimmen.

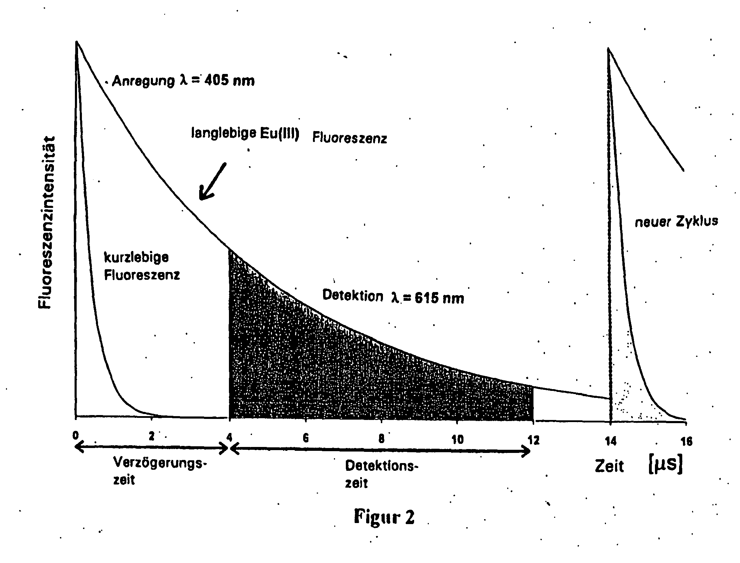

Eine quantitative Bestimmung kann durch Messung der Kinetik der Reaktion erfolgen, wie dies in Figur 2 dargestellt ist. Sie zeigt die Änderung der Emission über die Zeit als Funktion der in der Probe vorhandenen Aktivität der POx. Aus der Auftragung der Abnahme der Emission pro Zeiteinheit (Δl/Δt) gegen die zugegebene Menge an POx ergibt sich eine quantitative Beziehung.

Besondere Bedeutung hat das Verfahren für den einfachen Nachweis der Myeloperoxidase (MPOx). Über deren Bestimmung ist es möglich, akute myelocytische Leukämie von anderen Typen einer Leukämie zu unterscheiden. Der Test wurde bisher meist mit Reagenzien durchgeführt, die zur Bildung dunkel gefärbter Niederschläge (aus WP unter dem Einfluss der POx) führten. Dieser Nachteil tritt beim Einsatz des erfindungsgemäßen Verfahrens nicht mehr auf.

Weiterhin können erfindungsgemäß auch Substrate der Peroxidasen bestimmt werden. Peroxidasen akzeptieren WP als zweites Substrat. So wir z.B. Phenol durch Peroxidase (POx) nach folgender Reaktionsgleichung oxidiert:

2 C₆H₅ +H₂O₂ → C₁₂H₈(OH)₂ + 2 H₂O

(2 Phenol + WP → Bisphenol + 2 Wasser)

Über die Messung der verbrauchten Menge an WP kann man somit die Konzentration an Phenol ermitteln. Die Bestimmung erfolgt üblicherweise kinetisch, d.h. man verfolgt die Kinetik des Verbrauches von WP über die Zeit, typischerweise über 1 bis 5 Minuten.

Es ist aber auch möglich, andere Substrate zu bestimmen. Auch Substrate, die nicht direkt durch Katalasen oder Peroxidasen umgesetzt werden, sind einer Bestimmung mittels der erfindungsgemäßen Indikatoren zugänglich, nämlich dann, wenn das Substrat zuerst durch ein oder mehrere andere Enzyme abgebaut wird. Das aus dieser Umsetzung resultierende Produkt ist dann ein Substrat für eine Katalase oder Oxidase und wird von dieser unter Verbrauch von WP weiter abgebaut. Typische Enzymkaskaden dieser Art sind im Folgenden dargestellt:

Pospho-Tyrosin (+ Tyr-Kinase) → Tyrosin

Tyrosin (+ Tyrosinase) → DOPA

DOPA (+ POx) + EuTcWP → Dimer + EuTc

Somit kann man das Phosphotyrosin mittels eines erfindungsgemäßen Reagenzes unter Verwendung einer Peroxidase bestimmen. Das konkrete Beispiel ist vor allem wichtig im Zusammenhang mit der Bestimmung von Hemmern der Tyrosin-Kinase.

Das erfindungsgemäße Verfahren kann auch in Immunoassays eingesetzt werden. Katalasen und Peroxidasen werden in konjugierter Form in optischen Immuntests eingesetzt. In einem typischen Verfahren wird ein Antikörper mit einer Peroxidase markiert. Im Folgenden ist dieser Umstand durch einen Stern gekennzeichnet. Findet der so markierte Antikörper (Ak*) nun ein entsprechendes Antigen (Ag), so kommt es zur Bildung eines Ag/Ak*-Komplexes, der auch die Marker-POx enthält.

In einem typischen Sandwich-Assay wird zuerst ein Ak immobilisiert. An diesen wird ein Antigen Ag gebunden. An den entstandenen Ak/Ag-Komplex lässt man nun markierten Antikörper Ak* andocken:

Ak/Ag + Ak* → Ak/Ag/Ak*

Je mehr Ag zuerst gebildet worden ist, desto mehr Ak* wird nun sandwichartig an den ersten Komplex binden und das Konjugat Ak/AgAk* bilden. Wenn der Marker (*) eine POx war, kann man durch Bestimmung der Aktivität der POx im Ak/Ag/Ak*-Komplex die unbekannte Menge an Ag bestimmen. Erfindungsgemäß wird die Messung der Aktivität einer Katalase oder Peroxidase in einem Immunkompley ein Reagenz umassen Eu, einen organischen Liganden und WP, insbesondere das Reagenz EuTcWP, und im Falle der POx zusätzlich ein Substrat eingesetzt.

Durch Markierung einer DNA mit einer Katalase oder Peroxidase eröffnet sich - ähnlich wie beim Immuntest - die Möglichkeit des Nachweises einer eintretenden Hybridisierung und somit der Einsatz des erfindungsgemäßen Verfahrens in Hybridisierungsassays. Man weist die enzymatische Aktivität der an der DNA befindlichen Katalase bzw. Peroxidase mittels der . erfindungsgemäßen Reagenzien über das verbrauchte WP nach. Im Fall der Katalase braucht nur das Reagenz (z.B. EuTcWP), aber kein Zweitsubstrat zugegeben werden. Im Fall der Peroxidase ist ein Zweitsubstrat (z.B. Phenol) erforderlich. Man verfolgt die über die Zeit eintretende Abnahme der Fluoreszenzintensität oder Zunahme der Abklingzeit.

Bei den erfindungsgemäßen Verfahren zum Nachweis von Antigenen oder Nukleinsäure-Oligomeren wird das Reagenz in Schritt (c) bevorzugt als festes oder gelöstes Reagenz zugesetzt.

Die Erfindung wird durch die folgenden Beispiele und die beigefügten Figuren weiter erläutert.

Figur 1 zeigt die Absorptions- und Emissionsspektren des Komplexes aus Europium(III)-Ion, Tetracyclin und Wasserstoffperoxid (EuTcWP) vor bzw. nach dem Verbrauch des Wasserstoffperoxids durch eine Katalase bzw. eine Peroxidase. Der Verbrauch des Wasserstoffperoxids des EuTcWP führt zum Produkt EuTc. Dieses besitzt zum eine eine etwas stärkere Absorption bei 400 nm, zum anderen eine um das fast 15-fache schwächere Emissionsintensität bei ca. 615 nm.

Figur 2 gibt eine schematische Darstellung einer zeitaufgelösten Messung zur Unterdrückung einer Untergrundfluoreszenz. Man regt zur Zeit O mit einem kurzen Lichtpuls an, wartet danach das Abklingen der kurzlebigen Untergrundfluoreszenz ab. Nach dieser Verzögerungszeit ("delay time"; beim EuTcWP hier 4 µs) öffnet man das Detektionsfenster ("counting time") bis zur Zeit 12 µs und beginnt nach 14 s den gleichen Zyklus wieder. Diese Messtechnik hat den Vorteil einer praktisch vollständigen Unterdrückung der kurzlebigen Untergrundfluoreszenz, ist aber nur möglich, wenn die verwendeten Reagenzien eine deutlich langsam abklingendere Fluoreszenz als alle anderen Materialien haben.

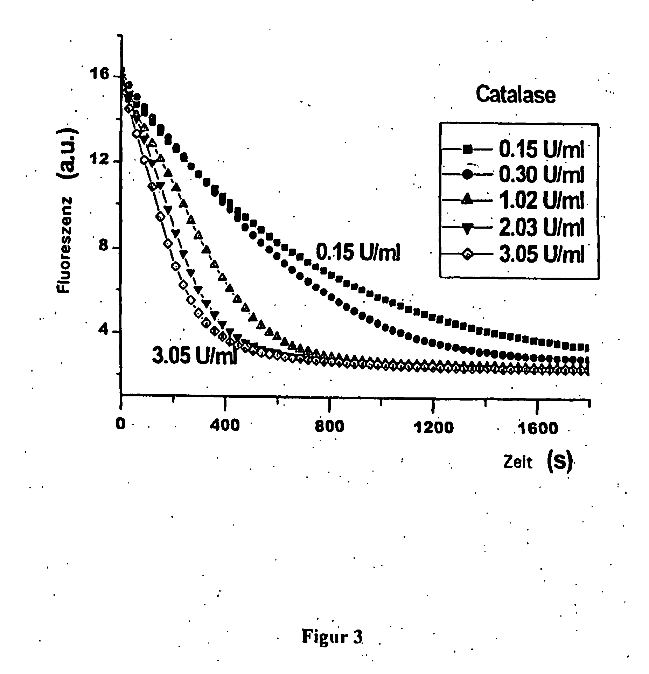

Figur 3: Enzymatischer Abbau des Komplexes aus Eu(III), Tetracyclin und Wasserstoffperoxid (Molverhältnis 17:51:400) durch zugegebene Katalase (aus Rinderleber). Der Komplex zerfällt unter Verbrauch von WP zum viel schwächer fluoreszierenden EuTc. Dies äußert sich in einer deutlichen Abnahme der Fluoreszenz bis einem Stand, der dem des EuTc-Komplexes entspricht. Je mehr Katalase zugegeben wird, desto schneller erfolgt der Zerfall. Die Abnahme der Fluoreszenz pro Zeiteinheit dient als Maß für die Aktivität des gelösten oder an einen Träger konjugierten Enzyms. Die Aktivität wird hier in Einheiten pro Milliliter (U/ml) angegeben.

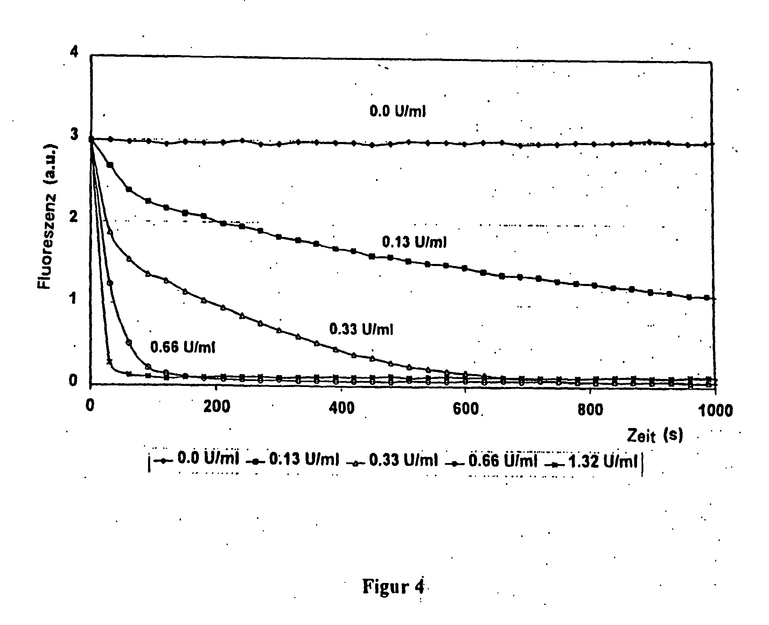

Figur 4: Enzymatischer Abbau des Komplexes, bestehend aus Eu(III), Tetracyclin und Wasserstoffperoxid ("EuTcWP") durch Zugabe des Enzyms Peroxidase und des Substrats Phenol. Aus dem EuTcWP-Komplex entsteht der nur schwach fluoreszierende wasserstoffperoxidfreie Komplex. Wieder dient die Abnahme der Fluoreszenz pro Zeiteinheit als Maß für die Aktivität (units/ml) des gelösten oder konjugierten Enzyms.

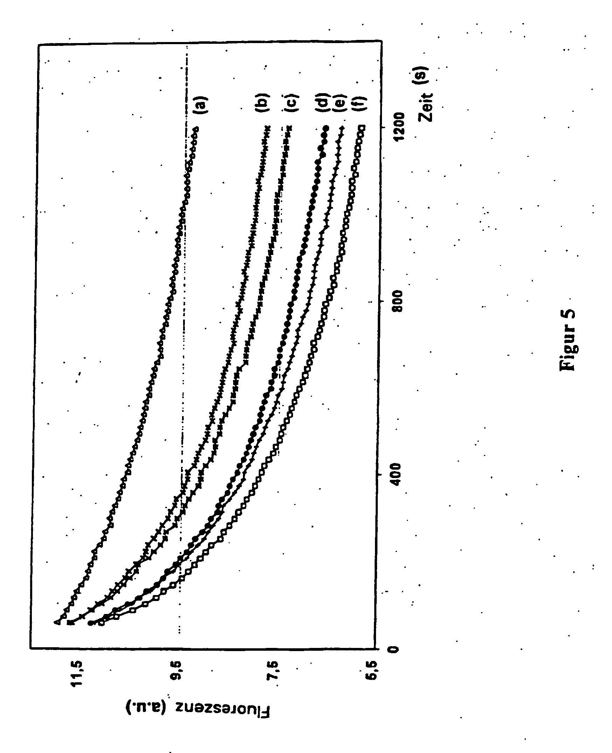

Figur 5 zeigt das Ergebnis eines ELISA-Assays. Das Antigen IgG wurde auf den Boden einer Mikrotiterplatte immobilisiert und mit verschiedenen Mengen an Anti-IgG umgesetzt, das mit POx markiert worden war. Nach Auswaschen und Zugabe des Reagenzes EuTcWP zersetzt die Peroxidase des Anti-IgG das Reagenz, was sich in einer deutlichen Abnahme der Fluoreszenzintensität äußert. Je mehr markiertes Anti-IgG zugegeben wurde, desto rascher erfolgte die Zersetzung. Kurve (a) zeigt den zeitlichen Verlauf in Abwesenheit von markiertem Anti-IgG. Dieser Beitrag muss als Blindwert abgezogen werden. Die zugegebenen Mengen an Anti-IgG-POx betrugen in (b) 0,3 ng/ml; in (c) 0,6 ng/ml; in (d) 3,0 ng/ml; in (e) 6,0 ng/ml; und in (f) 600 ng/ml.

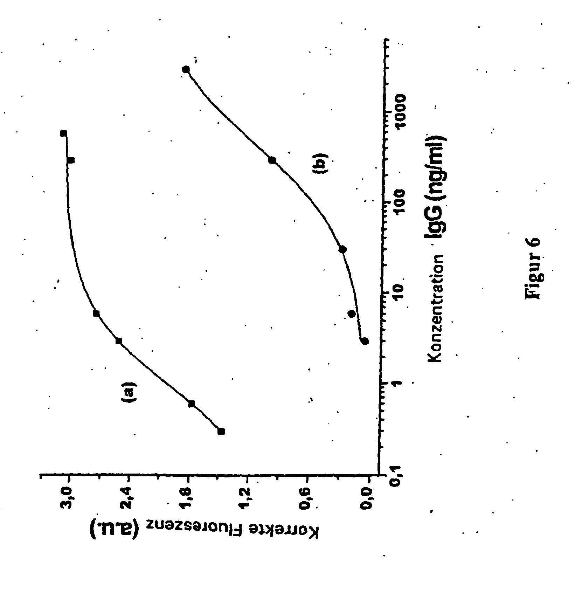

Figur 6 zeigt Kalibrationskurven, wie sie aus der Auftragung der korrigierten Fluoreszenz nach einer gewissen Zeit gegen die logarithmische Konzentration an IgG erhalten werden. Kurve (a): Kalibrationskurve eines Sandwich-ELISA; (b) Kalibrationskurve eines direkten ELISA. Der Sandwich-ELISA für das IgG ist etwa so empfindlich wie die empfindlichsten bekannten Nachweisverfahren (0,3 ng/ml).

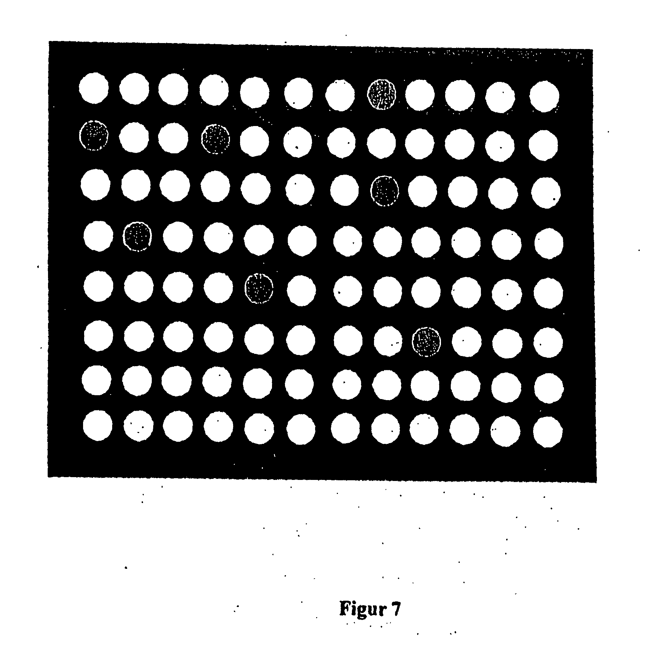

Figur 7 zeigt einen Ausschnitt aus einer 96-Mikrotiterplatte, in deren Vertiefungen ("wells") ein Target-Oligomer (siehe Beispiel 6) immobilisiert wurde. Danach wurden in jedes Well beliebige 20-mer Sequenzen zugegeben, in manche auch die exakt komplementäre E-HEC-Sequenz, die mit POx markiert worden war. Danach wurde hybridisiert, gewaschen und das Reagenz EuTcWP sowie Phenol zugegeben. In der gezeigten Figur stehen helle Zonen für starke Fluoreszenz. Diese tritt an jenen Stellen auf, an denen sich keine Peroxidase befindet, an denen also kein E-HEC-POx an das Target gebunden hatte. Dunkle Zonen zeigen an, dass sich an diesen Stellen POx-markiertes E-HEC befindet, welches das zugegebene fluoreszierende EuTcWP zum nur schwach fluoreszierenden EuTc abgebaut hatte.

### Beispiele

### Beispiel 1: Herstellung einer Reagenzlösung (EuTcWP)

Man löst 1,48 g des Puffersalzes MOPS Na⁺-Salz (Fluka AG) in 490 ml destilliertem Wasser, bringt den pH-Wert der Lösung mit wenigen Tropfen 70 %-iger Perchlorsäure auf pH 6,9 und füllt auf 500 ml auf. Die Reagenzlösung erhält man, indem man 4,0 mg Tetracyclin-Hydrochlorid (Fluka AG), 0,1 ml Wasserstoffperoxid (30 %-ige Lösung; Merck) und 9,6 mg EuCl₃-Hexahydrat (Alfa) in 100 ml des obigen Puffers löst. Das Reagens kann in trockener Form erhalten werden, indem man das gelöste Reagenz ohne Pufferzusatz herstellt und die Lösung danach gefriertrocknet. Andere Mengenverhältnisse zwischen Tetracyclin und Europium-Ion sind möglich.

### Beispiel 2: Bestimmung der Aktivität der Katalase

Diese Bestimmung erfolgt durch einfache Zugabe des Reagenzes nach Beispiel 1 zu einer Lösung, die zwischen 0,0 und 3,0 units/ml an Katalase enthält. Die Kinetik der Zersetzung des Wassrstoffperoxids im EuTcWP ist in Figur 3 dargestellt. Diese dient auch der quantitativen analytischen Auswertung.

### Beispiel 3: Enzymatische Bestimmung von Phenol

*(a) Peroxidase-Stammlösung:* Man löst 1,0 mg POx (aus Meerrettich; 1000 units/mg, Produkt P-6782; von Sigma) in 10 ml MOPS-Puffer.
*(b) Bestimmungsverfahren:* Man befüllt die Näpfe einer Mikrotiterplatte nacheinander mit folgenden Reagenzien: 0,2 ml der auf Phenol zu analysierenden Lösung (mit einem Gehalt zwischen 2 und 20 mg pro Liter), 0,01 ml der Reagenzlösung (siehe Beispiel 1) und schließlich mit 0,1 ml der Peroxidase-Stammlösung. Man verfolgt die Abnahme der Fluoreszenzintensität über die Zeit ab dem Zeitpunkt der Zugabe der POx. Der Anstieg der Fluoreszenz nach einer definierten Zeit, z.B. nach 3 min, ist ein Maß für die in der Probe vorhandene Konzentration an Phenol. Der genaue Wert kann anhand vorab ermittelter Kalibrationskurven berechnet werden.

Bei manchen Mikrotiterplatten-Lesegeräten ist auch eine zeitverzögerte Messung möglich. Dazu stellt man im Gerät (z.B. dem Ultra II; von TECAN; Crailsheim) eine Verzögerungszeit von 1 microsec ein und integriert danach die Emissionsintensität über 25 microsec, bevor der nächste Anregungspuls getriggert wird.

### Beispiel 4: Bestimmung von Enzymhemmern

Katalasen und Peroxidasen werden unter anderem durch Schwermetallionen, Sulfit- und Sulfid-Ionen massiv gehemmt (H. Zollner, Enzyme Inhibitors; Wiley & Sons, 1996). Die Menge an verbrauchtem WP ist von der Aktivität der Katalase bzw. POx abhängig und kann mit Hilfe eines erfindungsgemäßen Reagenzes bestimmt werden. Über die Verlangsamung der in Beispiel 2 beschriebenen Zersetzung des EuTcWP-Komplexes durch Katalase kann die Konzentration derartiger Hemmer bestimmt werden.

### Beispiel 5: Bestimmung eines Antigens (HSA) über einen Immunoassay auf magnetischen Partikeln

*(a) Biotinylierung von Anti-HSA:* Polyklonales Anti-HSA (Ziege; Sigma Prod. Nr. A-1151) wurde nach 10-facher Verdünnung mit Phosphatpuffer mit dem Biotinylierungsreagenz Biotinamidocapronsäure-sulfo-NHS-ester (Sigma, B-1022) nach der Vorschrift von Psantano & Kuhr in Analytical Chemistry 65 (1993) 623 umgesetzt. Das so biotinylierte Anti-HSA wurde danach an die Magnetpartikel gebunden (siehe unter (c)).
*(b) Markierung von Anti-HSA mit POx:* Die Markierung erfolgte über die Biotin-Streptavidin-Bindungsreaktion. Polyklonales Anti-HSA (Ziege; Sigma Prod. Nr. A-1151) wurde nach 10-facher Verdünnung mit Phosphatpuffer zuerst mit Streptavidin-Maleinimid (Sigma, Prod. Nr. S-9415) nach der Vorschrift von Duncan et al. in Analytical Biochemistry 132 (1983) 68 markiert. Das so entstandene Streptavidin/Anti-HSA-Konjugat wurde nach elektrophoresischer Aufreinigung mit dem POx-Markierungs-Reagenz Biotinamino-POx (Sigma Nr. P-9568) versetzt und elektrophoretisch gereinigt.
*(c) Immobilisierung von Anti-HSA auf Magnetbeads:* Die Immobilisierung erfolgte über die Biotin-Streptavidin-Bindungsreaktion. 1 ml einer Suspension von paramagnetischen Eisenoxidpartikeln mit oberflächlich gebundenem Streptavidin (d = 1 µm; Sigma; Prod. Nr. S 2415) enthaltend etwa 1 mg immobilisiertes Streptavidin) wurden mit 1 ml einer Lösung von biotinyliertem Anti-HSA (s. unter (a)) versetzt und 1 h bei Raumtemperatur stehen gelassen. Die Partikel wurden dann mit Hilfe eines Magnetseparators (Sigma, Prod. Nr. M-1292) abgetrennt.
*(d) Bestimmungsverfahren.* Die nach (c) erhaltenen Magnetpartikel wurden in 2 ml Phosphatpuffer von pH 7,0 suspendiert und mit Lösungen versetzt, die zwischen 2 und 20 µg/ml HSA enthielten, was der im Urin oder bei Mikroaluminurie vorkommende HSA-Konzentration entspricht. Nach 10 min wurden die Partikel mittels Magnetseparator abgetrennt, in 2 ml PBS resuspendiert und mit einer Lösung von 1 mg/ml des POx-markierten polyklonalen Anti-HSA (siehe (b)) versetzt. In einem derartigen Sandwich-Assay bindet das Anti-HSA an das bereits am Partikel befindliche HSA. Nach nochmaliger magnetischer Trennung und Resuspension wurden folgende Reagenzien zugegeben:
   100 ml einer 0,1 %-igen Lösung von Phenol in PBS;
   1 ml einer Lösung, die 17 µMol/l Tetracyclin (Sigma, Prod. Nr. T-3258), 51 µMol/l Europiumchlorid (Fluka) und 400 µMol/l Wasserstoffperoxid enthielt (ähnlich dem Reagenz unter Beispiel 1).

Die pro Zeiteinheit verbrauchte Menge an WP kann durch die Abnahme in der Fluoreszenz bei 610 bis 620 nm (unter Anregung bei 405 nm) bestimmt werden und ist proportional der in der Probe enthaltenen Konzentration an HSA.

### Beispiel 6: Nachweis einer spezifischen Oligomeren-Sequenz über einen Hybridisierungs-Assay

*(a) Prinzip:* Die Bindung eines POx-markierten 15-mers an ein komplementäres 15-mer, das auf ein Agarose-Partikel immobilisiert worden war, wurde über die mittel der erfindungsgemäßen Reagenzien nachweisbare Aktivität der POx nachgewiesen. Es wurde die Sequenz 5'-AAG-TAG-TCA-ACG-AAT-GGC-GA-3' ("E-HEC") untersucht, die für jenen E.coli-Mutanten (E-HEC) spezifisch ist, der für die gelegentlich durch infiziertes Rindfleisch verursachten Nahrungsmittelvergiftungen verantwortlich ist.
*(b) Herstellung eines mit Peroxidase markierten Oligomers*
   Ein 20-mer-Target der Sequenz 5'-AAG-TAG-TCA-ACG-AAT-GGC-GA-3' wurde am 5'-Ende mit Peroxidase markiert ("E-HEC-POx"). Das Komplementär-Oligo (5'-TCG-CCA-TTC-GTT-GAC-TAC-TT-3') wurde am 5'-Ende mit Biotin markiert (Metabion GmbH, München). Danach wurde das Komplementär ("COMP" an ein Streptavidin gebunden, das sich auf dem Boden einer Mikrotiterplatte befand.
*(c) Kompetitive Hybridisierung und Nachweis der Aktivität der POx am gebundenen Oligomer.* In jede Vertiefung der Mikrotiterplatte mit dem darin immobilisierten COMP wurden je 10 µl einer Lösung von Oligomer ("E-HEC") und von POx-markiertem Oligomer ("E-HEC-POx") eingetragen und bei 55 °C hybridisiert. Die Vertiefungen wurden entleert und gewaschen. Nach Zugabe von 20µl Reagenz (EuTcWP; siehe Beispiel 1) und 20µl einer Phenollösung wurde die Abnahme der Fluoreszenz bei 610 bis 620 nm (unter Anregung bei 405 nm) verfolgt. Die Abnahme der Fluoreszenz des EuTcWP war umso stärker, je weniger E-HEC vorhanden ist, da die meisten Bindungsstellen des Targets mit E-HEC-POx belegt worden waren. Bei Verwendung anderer POx-markierter Sequenzen als jener des E-HEC bzw. des E-HEX-POx tritt wegen nicht eintretender Hybridisierung keine wesentliche Abnahme der Fluoreszenz auf.

### Beispiel 7: Beispiel für ein zeitaufgelöstes Messverfahren für EuTcWP

Die Fluoreszenz der erfindungsgemäßen Europium-Reagenzien der allgemeinen Zusammensetzung EuTcWP klingt vergleichsweise langsam ab, aber deutlich schneller als z.B. Komplexe des Eu(III), wie sie im DELFIA-Immuntest als kovalente Marker eingesetzt werden. Das Messverfahren samt Zeitdomänen ist in Figur 2 dargestellt. Man regt die Fluoreszenz des Reagenzes mit einem kurzen Puls einer Xenonlampe (0,1 bis 1,5 µs), einer blauen (405 nm) oder einer UV-Leuchtdiode (375 nm; Nichia) an. Die Integration der emittierten Lichtintensität erfolgt dann nach einer Verzögerungszeit von 4 µs. Die Abklingzeit des EuTcWP liegt bei 9 µs, die des Reaktionsproduktes EuTc bei 13 µs.

## Patentansprüche

1. Verfahren zum qualitativen oder quantitativen Nachweis eines Analyten in einer Probe,
**dadurch gekennzeichnet,**
**dass** man
(a) einer Probe ein Reagenz zusetzt, das ein dreiwertiges Europium-Ion, mindestens einen organischen Liganden des Europium(III)-Ions und Wasserstoffperoxid enthält, worin die Probe einen Analyten enthält, der aus einem Enzym, einem an einem Biomolekül konjugierten Enzym, einem Enzyminhibitor, einem Enzymaktivator oder einem Substrat einer Peroxidase besteht, worin das Enzym aus einer Katalase oder einer Peroxidase besteht,
(b) der Probe, wenn eine Peroxidase nachgewiesen werden soll, ein Substrat der Peroxidase zusetzt; oder
(c) der Probe, wenn ein Substrat der Peroxidase nachgewiesen werden soll, eine Peroxidase zusetzt, und
(d) anhand von Änderungen der optischen Eigenschaften des in (a) eingesetzten Reagenzes den Analyten qualitativ nachweist oder quantitativ bestimmt.

2. Verfahren nach Anspruch 1,
**dadurch gekennzeichnet,**
**dass** das Enzym an ein Biomolekül oder an einen festen Träger konjugiert vorliegt.

3. Verfahren nach einem der Ansprüche 1 oder 2,
**dadurch gekennzeichnet,**
**dass** der Nachweis oder die quantitative Bestimmung in Blut, Serum, interstitieller Flüssigkeit, biologischem Gewebe, in Nahrungsmitteln oder Getränken, in pflanzlichen Produkten, in Bioreaktorflüssigkeiten, Zellen oder in Umweltproben durchgeführt wird.

4. Verfahren nach einem der vorhergehenden Ansprüche zum Nachweis oder zur Bestimmung von Hemmern der Katalase bzw. Peroxidase, **dadurch gekennzeichnet,**
**dass** der reaktionsverlangsamende Einfluss eines Enzymhemmers auf die durch eine Katalase oder Peroxidase bewirkte optische Änderung der Eigenschaften des zugesetzten Reagenzes gemessen und zum Nachweis der Anwesenheit oder zur Bestimmung der Konzentration des Hemmers herangezogen wird.

5. Verfahren nach einem der Ansprüche 1 bis 3 zum Nachweis oder zur Bestimmung von Hemmern des Enzyms,
**dadurch gekennzeichnet,**
**dass** es zum Screening auf die Wirksamkeit von potenziellen Pharmaka als Enzymhemmer eingesetzt wird, gegebenenfalls zusammen mit anderen Arten von Enzymen.

6. Verfahren nach einem der Ansprüche 1 bis 3 zum Nachweis oder zur Bestimmung von Aktivatoren des Enzyms,
**dadurch gekennzeichnet,**
**dass** der beschleunigende Einfluss eines Aktivators auf die Aktivität der Katalase bzw. Peroxidase bestimmt und zur Bestimmung dessen Konzentration herangezogen wird.

7. Verfahren nach Anspruch 1 zum qualitativen Nachweis oder zur quantitativen Bestimmung von Antigenen,
**dadurch gekennzeichnet,**
**dass** man
(a) einen Antikörper mit einer Katalase markiert;
(b) diesen Antikörper eine oder mehrere Immunbindungsreaktionen eingehen lässt,
(c) ein Reagenz zusetzt, das aus einem dreiwertigen Europium-Ion, zumindest einem organischen Liganden des Europium(III)-lons und aus Wasserstoffperoxid besteht,
(d) die durch die Marker-Katalase bewirkte Änderung der optischen, insbesondere fluoreszenzoptischen Eigenschaften des Reagenzes bestimmt, und
(e) über die eingetretene Änderung der optischen Eigenschaften des Reagenzes das Antigen qualitativ nachweist oder quantitativ bestimmt.

8. Verfahren nach Anspruch 1 zum qualitativen Nachweis oder zur quantitativen Bestimmung von Antigenen,
**dadurch gekennzeichnet,**
**dass** man
(a) einen Antikörper mit einer Peroxidase markiert;
(b) diesen Antikörper eine oder mehrere Immunbindungsreaktionen eingehen lässt,
(c) ein Reagenz zusetzt, das aus einem dreiwertigen Europium-Ion, einem organischen Liganden des Europium(III)-Ions und aus Wasserstoffperoxid besteht,
(d) ein Substrat der Peroxidase zusetzt,
(e) die durch die Marker-Katalase bewirkte Änderung der optischen, insbesondere fluoreszenzoptischen Eigenschaften des Reagenzes bestimmt, und
(f) über die eingetretene Änderung der optischen Eigenschaften des Reagenzes das Antigen qualitativ nachweist oder quantitativ bestimmt.

9. Verfahren zum qualitativen Nachweis oder zur quantitativen Bestimmung von Antigenen nach Anspruch 7 oder 8,
**dadurch gekennzeichnet,**
**dass** es ein Enzyme-linked immunosorbent assay (ELISA), vorzugsweise ein Sandwichassay ist.

10. Verfahren zum immunhistochemischen Nachweis von Antigenen nach Anspruch 7 oder 8,
**dadurch gekennzeichnet,**
**dass** ein mit einem Enzym markierter Antikörper an ein in einem Gewebe oder einem Gewebsabschnitt befindliches Antigen bindet und dass der Ort der Bindung durch Zugabe eines Reagenz, umfassend ein dreiwertige Europium-Ion, zumindest einen organischen Liganden des Europium(III)-Ions und Wasserstoffperoxid sichtbar gemacht wird.

11. Verfahren nach Anspruch 1 zum qualitativen Nachweis oder zur quantitativen Bestimmung von Nukleinsäure-Oligomeren,
**dadurch gekennzeichnet,**
**dass** man
(a) ein RNA-, DNA- oder PNA-Ollgomer mit einer Katalase markiert;
(b) das markierte Oligomer in ein RNA-, DNA- oder PNA-Hybridisierungsverfahren einsetzt,
(c) ein Reagenz zusetzt, das aus einem dreiwertigen Europium-Ion, zumindest einem organischen Liganden des Europium(III)-Ions und aus Wasserstoffperoxid besteht,
(d) die durch die Marker-Katalase bewirkte Änderung der optischen, insbesondere fluoreszenzoptischen Eigenschaften des Reagenzes bestimmt, und
(e) über die eingetretene Änderung der optischen Eigenschaften des Reagenzes das Oligomer qualitativ nachweist oder quantitativ bestimmt.

12. Verfahren nach Anspruch 1 zum qualitativen Nachweis oder zur quantitativen Bestimmung von Nukleinsäure-Oligomeren,
**dadurch gekennzeichnet,**
**dass** man
(a) ein RNA-, DNA oder PNA-Oligomer mit einer Peroxidase markiert;
(b) das markierte Oligomer in einem RNA-, DNA- oder PNA-Hybridisierungsverfahren einsetzt,
(c) ein Reagenzusetzt, das aus einem dreiwertigen Europium-Ion, zumindest einem organischen Liganden des Europium(III)-Ions und aus Wasserstoffperoxid besteht,
(d) ein Substrat der Peroxidase zusetzt,
(e) die durch die Marker-Peroxidase bewirkte Änderung der optischen, insbesondere fluoreszenzoptischen Eigenschaften des Reagenzes bestimmt und
(f) über die eingetretene Änderung der optischen Eigenschaften des Reagenzes das Oligomer qualitativ nachweist oder quantitativ bestimmt.

13. Verfahren zum qualitativen Nachweis oder zur quantitativen Bestimmung von Nukleinsäuren-Oligomeren nach Anspruch 11 oder 12, **dadurch gekennzeichnet,**
**dass** es zum Nachweis und zur quantitativen Bestimmung von Nukleinsäure-Oligomeren in Blut, Sperma, Speichel, Gewebe, Nahrungsmitteln, Pflanzen- oder Saatmaterial, Erbgut, Mikroorganismen oder Viren einsetzt.

14. Verfahren nach einem der vorhergehenden Ansprüche,
**dadurch gekennzeichnet,**
**dass** die verwendeten Biomoleküle auf einem flächigen Element, in einer Matrix, auf einer Mikrotiterplatte oder auf Partikeln immobilisiert vorliegen.

15. Verfahren nach einem der vorhergehenden Ansprüche,
**dadurch gekennzeichnet,**
**dass** eines oder mehrere der verwendeten Biomoleküle über das System (Strept)avidin/Biotin immobilisiert werden.

16. Reagenz der allgemeinen Zusammensetzung
Eu - Lig - (H₂O₂)
worin
Eu für das dreiwertige Europium-Ion,
Lig für zumindest einen organischen Liganden aus der Gruppe der β-Dicarbonylverbindungen bzw. deren Enole, und
H₂O₂ für Wasserstoffperoxid steht;
das molare Verhältnis von Eu zu Lig zwischen 30·1 und 1·5 und
das molare Verhältnis von Eu zu Wasserstoff peroxid zwischen 1:200 und 25:1 liegt, insbesondere in fester, gelöster oder immobilisierter Form für die Durchführung absorptiometrischer, reflektometrischer oder fluorimetrischer Verfahren zum Nachweis bzw. zur Bestimmung von gelösten, immobilisierten oder konjugierten Katalasen bzw. Peroxidasen, deren Substraten, Inhibitoren oder Aktivtoren.

17. Reagenz nach Anspruch 16 zur Durchführung eines bioanalytischen Verfahrens nach Anspruch 1 oder 2.

18. Reagenz nach Anspruch 16 oder 17,
**dadurch gekennzeichnet,**
**dass** der organische Ligand für Benzoylaceton, Benzoyltrifluoroaceton, Dibenzoylmethan, Thenoyltrifluoroaceton, heterocyclische (ortho-Hydroxy-)Carbonsäuren, aromatische oder heterocyclische ortho-Hydroxyketone und deren Derivate, Hydroxychinone und teilweise hydrierte und substituierte Hydroxychinon-artige Verbindungen einschließlich des Tetracyclins und seiner Derivate steht.

19. Verfahren zur Bestimmung von Antigenen oder Nukleinsäure-Oligomeren und deren Konjugate nach einem der Ansprüche 1 bis 15,
**dadurch gekennzeichnet,**
**das** die Visualisierung der Antigene bzw. Nukleinsäure-Oligomeren mit Hilfe sogenannter Blotting-Verfahren und unter Verwendung von Tranfermembranen erfolgt.

20. Verfahren nach einem der Ansprüche 1 bis 15,
**dadurch gekennzeichnet,**
**dass** es mit Hilfe eines automatisierten Fließ- bzw. Pipettiersystem durchgeführt wird, das eine mechanische Zuführung von Probenmaterial, Enzym (oder Enzymen) bzw. Enzym-markierten Biomolekülen und Reagenzien vorsieht.

21. Verfahren nach einem der Ansprüche 1 bis 15,
**dadurch gekennzeichnet,**
**dass** sich das Enzym bzw. das Enzym-markierte Biomolekül auf einem gegebenenfalls fluoreszent markierten Partikel mit einem Durchmesser von 0,1 bis 20 µm befinden.

22. Verfahren nach Anspruch 21,
**dadurch gekennzeichnet,**
**dass** die Partikel in fließ-cytometrischen Nachweisverfahren eingesetzt werden.

23. Verfahren nach einem der Ansprüche 21 oder 22,
**dadurch gekennzeichnet,**
**dass** die Partikel einen magnetischen Kern haben.

24. Verfahren nach einem der Ansprüche 1 bis 15,
**dadurch gekennzeichnet,**
**dass** man das Ausmaß der durch eine Katalase oder eine Peroxidase bewirkte Reaktion über die eingetretenen Änderungen in der Lichtabsorption des zugesetzten Reagenzes im Wellenlängenbereich zwischen 300 und 450 nm quantifiziert.

25. Verfahren nach einem der Ansprüche 1 bis 15,
**dadurch gekennzeichnet,**
**dass** man das Ausmaß der durch eine Katalase oder eine Peroxidase bewirkt Reaktion über die eintretenden Änderungen in der Lumineszenz des zugesetzten Reagenzes quantifiziert, indem man die Lösung mit Licht der Wellenlängen zwischen 300 und 450 nm bestrahlt und die Änderung in der Intensität oder der Abklingzeit der Emission bei Wellenlängen von > 550 nm ermittelt.

26. Verfahren nach einem der Ansprüche 1 bis 15,
**dadurch gekennzeichnet,**
**dass** man zur Unterdrückung der Eigenfluoreszenz des Untersuchungsmaterials bzw. des Systems die Lumineszenz des Reagenzes derart ermittelt, dass man zuerst ein Anregungslichtpuls mit einer Daur von < 20 µs einstrahlt und die Lumineszenz bei > 550 nm erst nach einer Verzögerungsphase von > 1 µs misst.

27. Verfahren nach einem der Ansprüche 1 bis 15,
**dadurch gekennzeichnet,**
**dass** man den durch die Katalase bzw. Peroxidase bewirkten Verbrauch von Wasserstoffperoxid über die pro Zeiteinheit eintretende Änderung der optischen Eigenschaften des Reagenzes verfolgt und diese mit der zu bestimmenden Größe in Relation setzt.

28. Verwendung eines Reagenz nach einem der Ansprüche 16 bis 18 zum qualitativen oder quantitativen Nachweis eines Enzyms, ausgewählt aus Katalasen oder Peroxidasen oder eine Substrats, Hemmers oder Aktivators davon.

29. Verfahren nach einem der Ansprüche 1-15 oder 19-27, worin die optische Eigenschaft Fluoreszenz ist.

## Claims

1. Method for the qualitative or quantitative detection of an analyte in a sample, **characterized in that**
(a) a reagent which contains a trivalent europium ion, at least one organic ligand of the europium(III) ion and hydrogen peroxide is added to a sample, wherein the sample contains an analyte which consists of an enzyme, an enzyme conjugated to a biomolecule, an enzyme inhibitor, an enzyme activator or a substrate of a peroxidase, wherein the enzyme consists of a catalase or a peroxidase,
(b) a substrate of peroxidase is added to the sample when a peroxidase is to be detected, or
(c) a peroxidase is added to the sample when a substrate of peroxidase is to be detected, and
(d) the analyte is qualitatively detected or quantitatively determined on the basis of changes in the optical properties of the reagent used in (a).

2. Method according to claim 1,
**characterized in that**
the enzyme is present conjugated to a biomolecule or to a solid carrier.

3. Method according to one of the claims 1 or 2,
**characterized in that**
the detection or the quantitative determination is carried out in blood, serum, interstitial fluid, biological tissue, in foods or drinks, in plant products, in biorector liquids, cells or in environmental samples.

4. Method according to one of the previous claims for the detection or determination of inhibitors of catalase or peroxidase,
**characterized in that**
the reaction-retarding effect of an enzyme inhibitor on the optical change in the properties of the added reagent caused by a catalase or peroxidase is measured and used to detect the presence or to determine the concentration of the inhibitor.

5. Method according to one of the claims 1 to 3 for detecting or determining inhibitors of the enzyme,
**characterized in that**
it is used to screen for the effectiveness of potential pharmaceutical agents as enzyme inhibitors, optionally together with other types of enzymes.

6. Method according to one of the claims 1 to 3 for detecting or determining activators of the enzyme,
**characterized in that**
the accelerating effect of an activator on the activity of catalase or peroxidase is determined and used to determine its concentration.

7. Method according to claim 1 for the qualitative detection or quantitative determination of antigens,
**characterized in that**
(a) an antibody is labelled with a catalase,
(b) this antibody is allowed to undergo one or more immunobinding reactions,
(c) a reagent is added which is composed of a trivalent europium ion, at least one organic ligand of the europium(III) ion and hydrogen peroxide,
(d) the change in the optical and in particular fluorescence-optical properties of the reagent caused by the catalase label is determined and
(e) the antigen is qualitatively detected or quantitatively determined by means of the change that occurs in the optical properties of the reagent.

8. Method according to claim 1 for the qualitative detection or quantitative determination of antigens,
**characterized in that**
(a) an antibody is labelled with a peroxidase,
(b) this antibody is allowed to undergo one or more immunobinding reactions,
(c) a reagent is added which is composed of a trivalent europium ion, an organic ligand of the europium(III) ion and hydrogen peroxide,
(d) a peroxidase substrate is added,
(e) the change in the optical and in particular fluorescence-optical properties of the reagent caused by the catalase label is determined and
(f) the antigen is qualitatively detected or quantitatively determined by means of the change that occurs in the optical properties of the reagent.

9. Method for the qualitative detection or the quantitative determination of antigens according to claim 7 or 8,
**characterized in that**
it is an enzyme-linked immunosorbent assay (ELISA), preferably a sandwich assay.

10. Method for the immunohistochemical detection of antigens according to claim 7 or 8,
**characterized in that**
an antibody labelled with an enzyme binds to an antigen located in a tissue or tissue section and the binding site is visualized by adding a reagent comprising a trivalent europium ion, at least one organic ligand of the europium(III) ion and hydrogen peroxide.

11. Method according to claim 1 for the qualitative detection or quantitative determination of nucleic acid oligomers,
**characterized in that**
(a) an RNA, DNA or PNA oligomer is labelled with a catalase,
(b) the labelled oligomer is used in an RNA, DNA or PNA hybridization procedure,
(c) a reagent is added which consists of a trivalent europium ion, at least one organic ligand of the europium(III) ion and hydrogen peroxide,
(d) the change in the optical and in particular fluorescence-optical properties of the reagent caused by the catalase label is determined and
(e) the oligomer is qualitatively detected or quantitatively determined by means of the change that occurs in the optical properties of the reagent.

12. Method according to claim 1 for the qualitative detection or quantitative determination of nucleic acid oligomers,
**characterized in that**
(a) an RNA, DNA or PNA oligomer is labelled with a peroxidase,
(b) the labelled oligomer is used in an RNA, DNA or PNA hybridization procedure,
(c) a reagent is added which consists a trivalent europium ion, at least one organic ligand of the europium(III) ion and hydrogen peroxide,
(d) a peroxidase substrate is added,
(e) the change in the optical and in particular fluorescence-optical properties of the reagent caused by the peroxidase label is determined and
(f) the oligomer is qualitatively detected or quantitatively determined by means of the change that occurs in the optical properties of the reagent.

13. Method for the qualitative detection or quantitative determination of nucleic acid oligomers according to claim 11 or 12,
**characterized in that**
it is used to detect or quantitatively determine nucleic acid oligomers in blood, sperm, saliva, tissue, foods, plant or seed material, genetic material, microorganisms or viruses.

14. Method according to one of the previous claims,
**characterized in that**
the biomolecules that are used are present immobilized on a planar element, in a matrix, on a microtitre plate or on particles.

15. Method according to one of the previous claims,
**characterized in that**
one or more of the said biomolecules are immobilized by means of the (strept)avidin/biotin system.

16. Reagent of the general composition
Eu - Lig - (H₂O₂)
in which
Eu represents a trivalent europium ion,
Lig represents at least one organic ligand from the group comprising β-dicarbonyl compounds or enols thereof and
H₂O₂ represents hydrogen peroxide;
the molar ratio of Eu to Lig is between 30:1 and 1:5 and
the molar ratio of Eu to hydrogen peroxide is between 1:200 and 25:1,
in particular in a solid, dissolved or immobilized form for carrying out absorptiometric, reflectometric or fluorimetric methods for detecting or determining dissolved, immobilized or conjugated catalases or peroxidases, or substrates, inhibitors or activators thereof.

17. Reagent according to claim 16 for carrying out a bioanalytical method according to claim 1 or 2.

18. Reagent according to claim 16 or 17,
**characterized in that**
the organic ligand represents benzoylacetone, benzoytrifluoroacetone, dibenzoylmethane, thenoyltrifluoroacetone, heterocyclic (ortho-hydroxy)-carboxylic acids, aromatic or heterocyclic ortho-hydroxyketones and derivatives thereof, hydroxyquinones and partially hydrogenated and substituted hydroxyquinone-like compounds including tetracycline and derivatives thereof.

19. Method for the determination of antigens or nucleic acid oligomers and conjugates thereof according to one of the claims 1 to 15, **characterized in that**
the antigens or nucleic acid oligomers are visualized with the aid of so-called blotting procedures and using transfer membranes.

20. Method according to one of the claims 1 to 15, **characterized in that**
it is carried out with the aid of an automated flow system or pipetting system which allows a mechanical addition of sample material, enzyme (or enzymes) or enzyme-labelled biomolecules and reagents.

21. Method according to one of the claims 1 to 15,
**characterized in that**
the enzyme or the enzyme-labelled biomolecule is present on an optionally fluorescently labelled particle having a diameter of 0.1 to 20 µm.

22. Method according to claim 21,
**characterized in that**
the particles are used in a flow-cytometric detection method.

23. Method according to one of the claims 21 or 22,
**characterized in that**
the particles have a magnetic core.

24. Method according to one of the claims 1 to 15,
**characterized in that**
the extent of the reaction caused by a catalase or peroxidase is quantified by means of the changes in the light absorption of the added reagent in the wavelength range between 300 and 450 nm.

25. Method according to one of the claims 1 to 15,
**characterized in that**
the extent of the reaction caused by a catalase or peroxidase is quantified by means of the changes in the luminescence of the added reagent by irradiating the solution with light of wavelengths between 300 and 450 nm and determining the change in the intensity or the decay time of the emission at wavelengths of > 550 nm.

26. Method according to one of the claims 1 to 15,
**characterized in that**
the self-fluorescence of the specimen material or the system is suppressed by determining the luminescence of the reagent in such a manner that it is firstly irradiated with an excitation light pulse lasting for < 20 µs and the luminescence at > 550 nm is not measured until after a delay phase of > 1 µs.

27. Method according to one of the claims 1 to 15,
**characterized in that**
the consumption of hydrogen peroxide caused by a catalase or peroxidase is monitored by means of the change in the optical properties of the reagent per unit of time and this is related to the parameter to be determined.

28. Use of a reagent according to one of the claims 16 to 18 for the qualitative or quantitative detection of an enzyme selected from catalases or peroxidases or a substrate, inhibitor or activator thereof.

29. Method according to one of the claims 1 - 15 or 19 - 27, wherein the optical property is fluorescence.

## Revendications

1. Procédé pour la mise en évidence qualitative ou quantitative d'un analyte dans un échantillon, **caractérisé en ce que**
(a) un ajoute à un échantillon un réactif qui contient un ion europium trivalent, au moins un ligand organique de l'ion europium (III) et du peroxyde d'hydrogène, où l'échantillon contient un analyte qui consiste en une enzyme, une enzyme conjuguée à une biomolécule, un inhibiteur d'enzyme, un activateur d'enzyme ou un substrat d'une peroxydase, où l'enzyme consiste en une catalase ou une peroxydase,
(b) quand une peroxydase doit être mise en évidence, on ajoute à l'échantillon un substrat de la peroxydase ; ou
(c) quand un substrat de peroxydase doit être mis en évidence, on ajoute à l'échantillon une peroxydase, et
(d) on met l'analyte en évidence qualitativement ou le détermine quantitativement à l'aide de modifications des propriétés optiques du réactif utilisé en (a).

2. Procédé selon la revendication 1 **caractérisé en ce que** l'enzyme est présente conjuguée à une biomolécule ou à un support solide.

3. Procédé selon l'une des revendications 1 et 2 **caractérisé en ce que** la mise en évidence ou la détermination quantitative est réalisée dans le sang, le sérum, le liquide interstitiel, un tissu biologique, dans des aliments ou des boissons, dans des produits végétaux, dans des liquides de bioréacteurs, des cellules ou dans des échantillons de l'environnement.

4. Procédé selon l'une des revendications précédentes pour la mise en évidence ou pour la détermination d'inhibiteurs de la catalase ou de la peroxydase, **caractérisé en ce que** l'influence ralentissant la réaction d'un inhibiteur d'enzyme sur la modification optique, due à une catalase ou une peroxydase, des propriétés du réactif ajouté est mesurée et utilisée pour la mise en évidence de la présence ou pour la détermination de la concentration de l'inhibiteur.

5. Procédé selon l'une des revendications 1 à 3 pour la mise en évidence ou pour la détermination d'inhibiteurs de l'enzyme **caractérisé en ce qu'**il est utilisé pour la sélection selon l'efficacité de produits pharmaceutiques potentiels comme inhibiteurs d'enzyme, éventuellement en même temps que d'autres types d'enzymes.

6. Procédé selon l'une des revendications 1 à 3 pour la mise en évidence ou pour la détermination d'activateurs de l'enzyme **caractérisé en ce que** l'influence accélératrice d'un activateur sur l'activité de la catalase ou de la peroxydase est déterminée et utilisée pour la détermination de sa concentration.

7. Procédé selon la revendication 1 pour la mise en évidence qualitative ou pour la détermination quantitative d'antigènes, **caractérisé en ce que**
(a) on marque un anticorps avec une catalase ;
(b) on fait participer cet anticorps à une ou plusieurs réactions d'immunoliaison,
(c) on ajoute un réactif qui consiste en un ion europium trivalent, au moins un ligand organique de l'ion europium (III) et en peroxyde d'hydrogène,
(d) on détermine la modification due au marqueur catalase des propriétés optiques, en particulier optiques de fluorescence, du réactif, et
(e) on met en évidence qualitativement ou on détermine quantitativement l'antigène par le biais de la modification des propriétés optiques du réactif qui est survenue.

8. Procédé selon la revendication 1 pour la mise en évidence qualitative ou pour la détermination quantitative d'antigènes, **caractérisé en ce que**
(a) on marque un anticorps avec une peroxydase ;
(b) on fait participer cet anticorps à une ou plusieurs réactions d'immunoliaison,
(c) on ajoute un réactif qui consiste en un ion europium trivalent, un ligand organique de l'ion europium (III) et en peroxyde d'hydrogène,
(d) on ajoute un substrat de la peroxydase,
(e) on détermine la modification due au marqueur catalase des propriétés optiques, en particulier optiques de fluorescence, du réactif, et
(f) on met en évidence qualitativement ou on détermine quantitativement l'antigène par le biais de la modification des propriétés optiques du réactif qui est survenue.

9. Procédé pour la mise en évidence qualitative ou pour la détermination quantitative d'antigènes selon la revendication 7 ou 8 **caractérisé en ce qu'**il s'agit d'un enzyme-linked immunosorbent assay (ELISA), de préférence d'un essai de type sandwich.

10. Procédé pour la mise en évidence immunohistochimique d'antigènes selon la revendication 7 ou 8, **caractérisé en ce qu'**un anticorps marqué avec une enzyme est lié à un antigène situé dans un tissu ou une section de tissu et **en ce que** le site de la liaison est rendu visible par addition d'un réactif, comprenant un ion europium trivalent, au moins un ligand organique de l'ion europium (III) et du peroxyde d'hydrogène.

11. Procédé selon la revendication 1 pour la mise en évidence qualitative ou pour la détermination quantitative d'oligomères d'acide nucléique **caractérisé en ce que**
(a) on marque un oligomère d'ARN, d'ADN ou d'APN avec une catalase ;
(b) on utilise l'oligomère marqué dans un procédé d'hybridation d'ARN, d'ADN ou d'APN,
(c) on ajoute un réactif qui consiste en un ion europium trivalent, au moins un ligand organique de l'ion europium (III) et en peroxyde d'hydrogène,
(d) on détermine la modification due au marqueur catalase des propriétés optiques, en particulier des propriétés optiques de fluorescence, du réactif, et
(e) on met en évidence qualitativement ou on détermine quantitativement l'oligomère par le biais de la modification des propriétés optiques du réactif qui est survenue.

12. Procédé selon la revendication 1 pour la mise en évidence qualitative ou pour la détermination quantitative d'oligomères d'acide nucléique **caractérisé en ce que**
(a) on marque un oligomère d'ARN, d'ADN ou d'APN avec une peroxydase ;
(b) on utilise l'oligomère marqué dans un procédé d'hybridation d'ARN, d'ADN ou d'APN,
(c) on ajoute un réactif qui consiste en un ion europium trivalent, au moins un ligand organique de l'ion europium (III) et en peroxyde d'hydrogène,
(d) on ajoute un substrat de la peroxydase,
(e) on détermine la modification due au marqueur peroxydase des propriétés optiques, en particulier des propriétés optiques de fluorescence, du réactif, et
(f) on met en évidence qualitativement ou on détermine quantitativement l'oligomère par le biais de la modification des propriétés optiques du réactif qui est survenue.

13. Procédé pour la mise en évidence qualitative ou pour la détermination quantitative d'oligomères d'acide nucléique selon la revendication 11 ou 12 **caractérisé en ce qu'**il est utilisé pour la mise en évidence et pour la détermination quantitative d'oligomères d'acide nucléique dans le sang, le sperme, la salive, les tissus, les aliments, le matériel végétal ou de semences, le patrimoine héréditaire, les micro-organismes ou les virus.

14. Procédé selon l'une des revendications précédentes **caractérisé en ce que** les biomolécules utilisées sont présentes immobilisées sur un élément plan, dans une matrice, sur une microplaque de titrage ou sur des particules.

15. Procédé selon l'une des revendications précédentes **caractérisé en ce qu'**une ou plusieurs des biomolécules utilisées sont immobilisées par le biais du système (strept)avidine/biotine.

16. Réactif de composition générale
Eu - Lig - (H₂O₂)
où
Eu représente l'ion europium trivalent,
Lig représente au moins un ligand organique du groupe des composés β-dicarbonylés ou de leurs endols, et
H₂O₂ représente le peroxyde d'hydrogène ;
le rapport molaire de Eu à Lig est entre 30 : 1 et 1 : 5 et
le rapport molaire de Eu au peroxyde d'hydrogène est entre 1 : 200 et 25 : 1,
en particulier sous forme solide, dissoute ou immobilisée pour la mise en oeuvre de procédés absorptiométriques, réflectométriques ou fluorimétriques pour la mise en évidence ou pour la détermination de catalases ou peroxydases dissoutes, immobilisées ou conjuguées, de leurs substrats, inhibiteurs ou activateurs.

17. Réactif selon la revendication 16 pour la mise en oeuvre d'un procédé bioanalytique selon la revendication 1 ou 2.

18. Réactif selon la revendication 16 ou 17 **caractérisé en ce que** le ligand organique représente la benzoylacétone, la benzoyltrifluoroacétone, le dibenzoylméthane, la thénoyltrifluoroacétone, des acides (ortho-hydroxy)carboxyliques hétérocycliques, des orthohydroxycétones aromatiques ou hétérocycliques et leurs dérivés, des hydroxyquinones et des composés de type hydroxyquinone partiellement hydrogénés et substitués y compris la tétracycline et ses dérivés.

19. Procédé pour déterminer des antigènes ou des oligomères d'acide nucléique et leurs conjugués selon l'une des revendications 1 à 15 **caractérisé en ce que** la visualisation des antigènes ou des oligomères d'acide nucléique a lieu à l'aide de procédés dits de blotting et au moyen de membranes de transfert.

20. Procédé selon l'une des revendications 1 à 15 **caractérisé en ce qu'**il est mis en oeuvre à l'aide d'un système de flux ou de pipetage automatisé qui prévoit un apport mécanique de matériel d'échantillon, d'enzyme (ou d'enzymes) ou de biomolécules marquées avec une enzyme et de réactifs.

21. Procédé selon l'une des revendications 1 à 15 **caractérisé en ce que** l'enzyme ou la biomolécule marquée avec une enzyme se trouve sur une particule marquée éventuellement par fluorescence d'un diamètre de 0,1 à 20 µm.

22. Procédé selon la revendication 21 **caractérisé en ce que** les particules sont utilisées dans un procédé de mise en évidence par cytométrie de flux.

23. Procédé selon l'une des revendications 21 et 22 **caractérisé en ce que** les particules ont un noyau magnétique.

24. Procédé selon l'une des revendications 1 à 15 **caractérisé en ce que** l'on quantifie l'ampleur de la réaction due à une catalase ou une peroxydase par le biais des modifications survenues dans l'absorption lumineuse du réactif ajouté dans le domaine de longueurs d'onde entre 300 et 450 nm.

25. Procédé selon l'une des revendications 1 à 15 **caractérisé en ce que** l'on quantifie l'ampleur de la réaction due à une catalase ou une peroxydase par le biais des modifications survenues dans la luminescence du réactif ajouté par le fait que l'on irradie la solution avec de la lumière de longueurs d'onde entre 300 et 450 nm et on détermine la modification de l'intensité ou du temps de décroissance de l'émission à des longueurs d'onde > 550 nm.

26. Procédé selon l'une des revendications 1 à 15 **caractérisé en ce que** pour réprimer la fluorescence propre du matériel d'examen ou du système, on détermine la luminescence du réactif de telle manière que l'on émet d'abord une impulsion de lumière d'excitation d'une durée < 20 µs et on mesure la luminescence à > 550 nm seulement après une phase de retard de > 1 µs.

27. Procédé selon l'une des revendications 1 à 15 **caractérisé en ce que** l'on suit la consommation de peroxyde d'hydrogène due à la catalase ou à la peroxydase par le biais de la modification survenue par unité de temps des propriétés optiques du réactif et on met celle-ci en relation avec la grandeur à déterminer.

28. Utilisation d'un réactif selon l'une des revendications 16 à 18 pour la mise en évidence qualitative ou quantitative d'une enzyme, choisie parmi les catalases ou les peroxydases ou un substrat, un inhibiteur ou un activateur de celles-ci.

29. Procédé selon l'une des revendications 1-15 ou 19-27 où la propriété optique est la fluorescence.
